# EUROPÄISCHE PATENTSCHRIFT

(11) **EP 2 746 762 B1**
(45) Veröffentlichungstag und Bekanntmachung des Hinweises auf die Patenterteilung: **22.01.2020**
(21) Anmeldenummer: 12198882.8
(22) Anmeldetag: 21.12.2012
(51) Int. Cl.: G01N 33/00, G01N 21/53

(54) **Tunnelüberwachungssensor**
Tunnel monitoring sensor
Capteur de surveillance de tunnel

(43) Veröffentlichungstag der Anmeldung: 25.06.2014
(73) Patentinhaber: SICK AG, 79183 Waldkirch/Breisgau (DE)
(72) Erfinder: Bönisch, Andreas, 79194 Gundelfingen (DE); Convent, Jürgen, 79183 Waldkirch (DE)
(74) Vertreter: Manitz Finsterwald Patent- und Rechtsanwaltspartnerschaft mbB

(56) Entgegenhaltungen:
- EP-A1- 0 240 713
- DE-A1- 10 142 711
- KR-A- 20030 067 886
- US-A- 5 349 267
- US-B1- 6 724 917
- CODEL International Ltd.: "TunnelCraft 3 - Air Quality Monitor CO,NO & Visibility, Product Data Sheet", , 9. November 2010 (2010-11-09), XP055059190, Gefunden im Internet: URL:http://www.pogc.com.au/pdf/pogc/CODEL/ TunnelCraftIIIAirQualityMonitorVisibility. pdf [gefunden am 2013-04-10]
- Sick Maihak Gmbh: "VICOTEC450 Sichttrübungsmessung - Produktinformation", , 1 May 2006 (2006-05-01), XP055377551, Retrieved from the Internet: URL:http://www.nuovaelva.it/files/docs/Sic k/ANALISI/Vari/Tunnel/VICOTEC450/PI_Vicote c450_de_2006-05_8011720.pdf [retrieved on 2017-05-31]
- Sigrist: "VISIBILITY MONITOR VISGUARD - data sheet", , 1 February 2011 (2011-02-01), XP055377710, Retrieved from the Internet: URL:https://www.photometer.com/svc/documen t.axd?id=8502&hl=E [retrieved on 2017-05-31]

## Beschreibung

Die vorliegende Erfindung betrifft einen Tunnelüberwachungssensor zum Überwachen von Umweltbedingungen in einem Tunnel mit einem ersten Sensormodul, welches ein Sichttrübungssignal ausgibt, und einem zweiten Sensormodul, welches ein erstes Gaskonzentrationssignal ausgibt.

Solche Sensoren werden üblicherweise im Inneren von Tunneln montiert, um den aktuellen Zustand der Umgebungsluft im Tunnel zu erfassen. Insbesondere in Straßen- und Schienenverkehrstunneln ist es aus Sicherheitsgründen unerlässlich, bestimmte Ereignisse wie Nebelbildung, Rauchentwicklung, Feinstaubbelastung oder erhöhte Abgaskonzentrationen rechtzeitig zu erkennen. Üblicherweise erfolgt die Überwachung der verschiedenen Messgrößen anhand mehrerer in dem Tunnel montierter Sensoren, deren Signale einer zentralen Steuereinrichtung, welche sich beispielsweise in einer Tunnelwarte befindet, zugeführt werden. In der Steuereinrichtung werden die Signale aller vorhandenen Sensoren ausgewertet. Anhand der Auswertung werden bedarfsweise Maßnahmen eingeleitet, um auf bestimmte Störungsereignisse zu reagieren . Beispielsweise kann bei einem Anstieg der Abgaskonzentration die Lüfterdrehzahl eines im Tunnel installierten Ventilationssystems erhöht werden oder im Falle einer Rauchentwicklung eine Tunnelsperrung ausgelöst werden.

Im Allgemeinen ist es aufgrund von geltenden Sicherheitsvorschriften notwendig, im Falle einer Wartung der Sensoren den Verkehr im Tunnel zumindest teilweise zu unterbrechen. Dies ist für den Tunnelbetreiber in der Regel mit hohen Kosten verbunden. Bei der Montage eines neuen Sensors oder beim Austausch eines defekten Sensors kann sich die Sperrzeit noch dadurch verlängern, dass vor Ort ein Abgleich des neuen Sensors durchgeführt werden muss. Ein weiteres Problem bei der Überwachung von Umweltbedingungen in Tunneln besteht darin, dass Fehlalarme oft besonders kostspielig sind, da sie mit weitreichenden Konsequenzen wie einer Tunnelsperrung oder einem längeren Betrieb des Ventilationssytems mit erhöhter Lüfterdrehzahl und dementsprechend vermehrten Stromkosten verbunden sind.

In dem Datenblatt von CODEL International Ltd.: "Tunnelcraft 3 - Air Quality Monitor CO, NO & Visibility, Product Data Sheet" ist ein Tunnelüberwachungssensor offenbart, welcher eine Sende-/ Empfangseinheit für sichtbares Licht und Infrarotlicht sowie einen von der Sende-/ Empfangseinheit beabstandet im Tunnel zu montierenden Reflektor umfasst. Anhand der Lichtabsorption werden die Sichtverhältnisse im Tunnel sowie die Konzentrationen von CO und NO ermittelt.

Die KR 2003 0067886 A offenbart einen Tunnelüberwachungssensor, der sowohl die allgemeinen Sichtverhältnisse als auch die Konzentration von Kohlenmonoxid anhand der Schwächung von Lichtstrahlen im Tunnel ermittelt.

Die EP 0 240 713 A1 offenbart ein Steuergerät für ein Tunnelbelüftungssystem, welches eine Vielzahl von Sensoreingaben verarbeitet.

In der DE 101 42 711 A1 ist eine Sensoranordnung zum Ansteuern einer Kraftfahrzeug-Klimaanlage offenbart, mittels welcher sowohl die Luftgüte als auch die Ozonkonzentration gemessen wird.

Es ist eine Aufgabe der Erfindung, einen Tunnelüberwachungssensor bereitzustellen, welcher einfach zu montieren und zu warten ist und welcher eine erhöhte Zuverlässigkeit aufweist.

Die Lösung der Aufgabe erfolgt durch einen Tunnelüberwachungssensor mit den Merkmalen des Anspruchs 1.

Erfindungsgemäß umfasst ein Tunnelüberwachungssensor ein gemeinsames Gehäuse für das erste und das zweite Sensormodul sowie eine vorzugsweise in dem Gehäuse untergebrachte elektronische Verknüpfungseinheit, welche das Sichttrübungssignal und das Gaskonzentrationssignal empfängt und aufgrund einer Verknüpfung des Sichttrübungssignals und des Gaskonzentrationssignals ein kombiniertes Umweltbedingungssignal ausgibt.

Die Erfindung sieht vor, dass das kombinierte Umweltbedingungssignal bei einem Anstieg des Sichttrübungssignals ohne gleichzeitigen Anstieg des ersten Gaskonzentrationssignals das Vorliegen von Nebel oder einer Feinstaubverschmutzung angibt und/oder das kombinierte Umweltbedingungssignal bei einem schnellen Anstieg des Sichttrübungssignals und gleichzeitig des ersten Gaskonzentrationssignals einen Brand angibt.

Es kann ferner vorgesehen sein, dass das erste Sensormodul vollständig innerhalb des Gehäuses angeordnet ist, wobei das Gehäuse wenigstens eine Lufteintrittsöffnung aufweist. Durch die Lufteintrittsöffnung kann die Umgebungsluft des Tunnelüberwachungssensors in das Gehäuse gelangen, so dass das erste Sensormodul z.B. anhand der Lichtstreuung über eine vorbestimmte Strecke die Sichttrübung ermitteln kann.

Es werden also wenigstens zwei unterschiedliche Sensorsignale gebündelt und in Form eines gemeinsamen Umweltbedingungssignals ausgegeben. Dadurch, dass die beiden Sensormodule in ein gemeinsames Gehäuse integriert sind, ist die Installation gegenüber einer Anordnung aus zwei separaten Sensormodulen erleichtert. Da bereits von dem Tunnelüberwachungssensor selbst eine Verknüpfung des Sichttrübungssignals und des gleichzeitig bereitgestellten Gaskonzentrationssignals durchgeführt wird, kann insgesamt eine differenziertere Auswertung erfolgen, so dass das Risiko für Fehlalarme verringert wird. Insbesondere kann bei der Erkennung von Störungsereignissen bereits sensorseitig sowohl die Sichttrübung als auch die Konzentration eines bestimmten Gases berücksichtigt werden. Beispielsweise könnte bei einem gleichzeitigen Ansteigen des Sichttrübungssignals und des Gaskonzentrationssignals ein Umweltbedingungssignal ausgegeben werden, welches auf einen Stau im Tunnel hinweist. In der Tunnelwarte kann das auf einer solchen Vorauswertung beruhende kombinierte Umweltbedingungssignal dann einer weitergehenden Auswertung unterzogen werden. Es ist jedoch auch möglich, das kombinierte Umweltbedingungssignal direkt, d.h. ohne nachträgliche Auswertung, zu verwenden, z.B. indem ein erfindungsgemäßer Tunnelüberwachungssensor direkt an ein Steuergerät des Ventilationssystems angeschlossen wird. In beiden Fällen ermöglicht das kombinierte Umweltbedingungssignal ein effektiveres Einleiten von Maßnahmen gegen Störungsereignisse als es unter Verwendung herkömmlicher Einzelsensoren möglich wäre.

Weiterbildungen der Erfindung sind in den abhängigen Ansprüchen, der Beschreibung sowie den beigefügten Zeichnungen angegeben.

Gemäß einer Ausführungsform der Erfindung sind das erste Sensormodul und das zweite Sensormodul zum Ausgeben jeweiliger digitaler Signale ausgebildet, wobei die elektronische Verknüpfungseinheit aufgrund einer logischen Verschaltung des digitalen Sichttrübungssignals und des digitalen Gaskonzentrationssignals ein kombiniertes digitales Umweltbedingungssignal ausgibt. Durch eine derartige logische Verknüpfung wenigstens zweier digitaler Messsignale kann eine besonders zuverlässige Klassifizierung der aktuellen Umweltbedingungen im Tunnel erzielt werden.

Die logische Verschaltung kann hierbei eine Mustererkennung beinhalten. Dies ermöglicht insbesondere bei Vorhandensein einer Vielzahl von Sensorsignalen eine besonders exakte Klassifizierung von Umweltbedingungen bzw. von Störungsereignissen.

Vorzugsweise gibt die elektronische Verknüpfungseinheit das kombinierte digitale Umweltbedingungssignal über eine digitale Standard-Schnittstelle, insbesondere über eine PROFIBUS-Schnittstelle, aus. Dies ist vor allem insofern von Vorteil, als nur ein einziges Kabel zur Übermittlung aller gewünschten Sensorsignale notwendig ist und sich die Installation insgesamt besonders einfach gestaltet. Die einzelnen Sensormodule des Tunnelüberwachungssensors können über interne Bus-Schnittstellen oder über eine serielle Schnittstelle mit der elektronischen Verknüpfungseinheit verbunden sein.

Gemäß einer weiteren Ausführungsform der Erfindung umfasst ein Tunnelüberwachungssensor wenigstens ein weiteres Sensormodul, welches ein weiteres Gaskonzentrationssignal ausgibt, wobei sich das erste Gaskonzentrationssignal und das weitere Gaskonzentrationssignal auf unterschiedliche Zielgase beziehen und die elektronische Verknüpfungseinheit das kombinierte Umweltbedingungssignal aufgrund einer Verknüpfung des Sichttrübungssignals, des ersten Gaskonzentrationssignals und des weiteren Gaskonzentrationssignals ausgibt. Je nach Anwendung können auch noch zusätzliche Sensormodule für weitere Zielgase vorgesehen sein. Auf diese Weise ist es möglich, die Konzentrationen unterschiedlicher gefährlicher Gase wie Kohlenmonoxid, Stickstoffmonoxid und Stickstoffdioxid bei der Beurteilung der Umweltbedingungen in dem Tunnel zu berücksichtigen. Eine durch die elektronische Verknüpfungseinheit auslesbare Kennung des jeweiligen Sensormoduls kann dabei eine eindeutige Zuordnung zwischen dem betreffenden Gaskonzentrationssignal und dem Zielgas ermöglichen. Somit kann der Tunnelüberwachungssensor bei Bedarf leicht mit Sensormodulen für zusätzliche Zielgase nachgerüstet werden.

Eine spezielle Ausgestaltung der Erfindung sieht vor, dass das zweite Sensormodul an einem Wandabschnitt des Gehäuses angebracht ist, wobei ein Messkopf des zweiten Sensormoduls durch eine Durchführung des Wandabschnitts hindurch in den Außenraum reicht. Der Messkopf kann somit direkt die Gaskonzentration in der Umgebungsluft des Tunnelüberwachungssensors erfassen. Grundsätzlich kann die Erfassung der Gaskonzentration durch den Messkopf mittels Molekülabsorption des transmittierten Lichts bei einer vorgegebenen Wellenlänge, mittels einer elektrochemischen Zelle oder mittels eines Halbleiterelements erfolgen.

Vorzugsweise umfasst das erste Sensormodul einen Streulichtsensor. Streulichtsensoren beruhen auf der Erfassung des in einem bestimmten Winkel zur Abstrahlungsrichtung einer Lichtquelle abgelenkten Lichtanteils und ermöglichen eine zuverlässige Erkennung der aktuellen Sichttrübung.

Eine weitere Ausführungsform der Erfindung sieht wenigstens ein weiteres Sensormodul vor, welches ein Temperatursignal und/oder ein Feuchtesignal ausgibt, wobei die elektronische Verknüpfungseinheit das kombinierte Umweltbedingungssignal aufgrund einer Verknüpfung des Sichttrübungssignals, des ersten Gaskonzentrationssignals und zusätzlich des Temperatursignals und/oder des Feuchtesignals ausgibt. Dies ermöglicht die Berücksichtigung der Temperatur und der Luftfeuchte im Tunnel beim Beurteilen der Umweltbedingungen. Beispielsweise kann die elektronische Verknüpfungseinheit bei einem gleichzeitigen Anstieg des Sichttrübungssignals und des Feuchtesignals sowie gleich bleibendem Gaskonzentrationssignal und gleich bleibendem Temperatursignal ein "Nebel"-Signal ausgeben. Bei einem Anstieg des Sichttrübungssignals ohne gleichzeitigen Anstieg des Gaskonzentrationssignals, des Feuchtesignals und des Temperatursignals kann die elektronische Verknüpfungseinheit hingegen ein "Feinstaub"-Signal ausgeben. Insbesondere kann als weiteres Sensormodul ein Halbleitersensor vorgesehen sein, welcher sowohl die Feuchte als auch die Temperatur erfasst.

Weiterhin kann die elektronische Verknüpfungseinheit zur Ausgabe eines Fehlersignals ausgebildet sein, welches einen Funktionsstatus des ersten Sensormoduls und/oder des zweiten Sensormoduls angibt. Eine Fehlfunktion oder ein Ausfall eines der Sensormodule kann somit rechtzeitig erkannt werden, z.B. in der zugehörigen Tunnelwarte. Dies erhöht die Sicherheit der Gesamtanlage.

Eine weitere Ausführungsform der Erfindung sieht vor, dass das erste Sensormodul und/oder das zweite Sensormodul eine Speichereinrichtung umfasst/umfassen, in welcher Kalibrierparameter des betreffenden Sensormoduls abgelegt sind. Somit erübrigen sich bei einer Neuinstallation oder bei einem Austausch eines Sensormoduls aufwändige Abgleich- und Parametriervorgänge, da das neue Sensormodul bereits alle notwendigen Parameter bereithält.

Gemäß einer weiteren Ausführungsform der Erfindung umfasst/umfassen das erste Sensormodul und/oder das zweite Sensormodul einen Mikroprozessor, welcher insbesondere zur Ausführung eines Selbsttests des betreffenden Sensormoduls ausgebildet ist. Die Sensormodule sind also gewissermaßen mit einer eigenen Intelligenz ausgestattet, so dass die Zuverlässigkeit des Tunnelüberwachungssensors weiter gesteigert werden kann.

An dem Gehäuse kann ferner wenigstens ein Anschluss für ein außerhalb des Gehäuses befindliches Zusatzsensormodul vorgesehen sein. Somit können nicht nur die in das Gehäuse integrierten Sensormodule, sondern bei Bedarf auch separate Sensormodule ausgewertet werden, um so ein weiter differenziertes kombiniertes Umweltbedingungssignal zu erzeugen.

Die Erfindung betrifft auch ein Tunnelüberwachungsverfahren zum Überwachen von Umweltbedingungen in einem Tunnel, bei welchem mittels eines ersten, in einem Gehäuse angeordneten Sensormoduls ein Sichttrübungssignal erzeugt wird, mittels eines zweiten in dem Gehäuse angeordneten Sensormoduls ein erstes Gaskonzentrationssignal erzeugt wird, das Sichttrübungssignal und das Gaskonzentrationssignal miteinander verknüpft werden und aufgrund der Verknüpfung ein kombiniertes Umweltbedingungssignal erzeugt wird, welches bei einem Anstieg des Sichttrübungssignals ohne gleichzeitigen Anstieg des ersten Gaskonzentrationssignals das Vorliegen von Nebel oder einer Feinstaubverschmutzung angibt und/oder bei einem schnellen Anstieg des Sichttrübungssignals und gleichzeitig des ersten Gaskonzentrationssignals einen Brand angibt. Vorteile und bevorzugte Ausgestaltungen eines erfindungsgemäßen Tunnelüberwachungsverfahrens ergeben sich aus den vorstehend genannten Vorteilen und bevorzugten Ausgestaltungen eines erfindungsgemäßen Tunnelüberwachungssensors.

Die Erfindung wird nachfolgend beispielhaft unter Bezugnahme auf die Zeichnungen beschrieben.
- Fig. 1: ist eine Draufsicht auf einen Tunnelüberwachungssensor gemäß einer ersten Ausführungsform der Erfindung.
- Fig. 2: zeigt den Tunnelüberwachungssensor gemäß Fig. 1 in einer teilweise aufgeschnittenen Darstellung.
- Fig. 3: zeigt einen Tunnelüberwachungssensor gemäß einer zweiten Ausführungsform der Erfindung in einer teilweise aufgeschnittenen Darstellung.

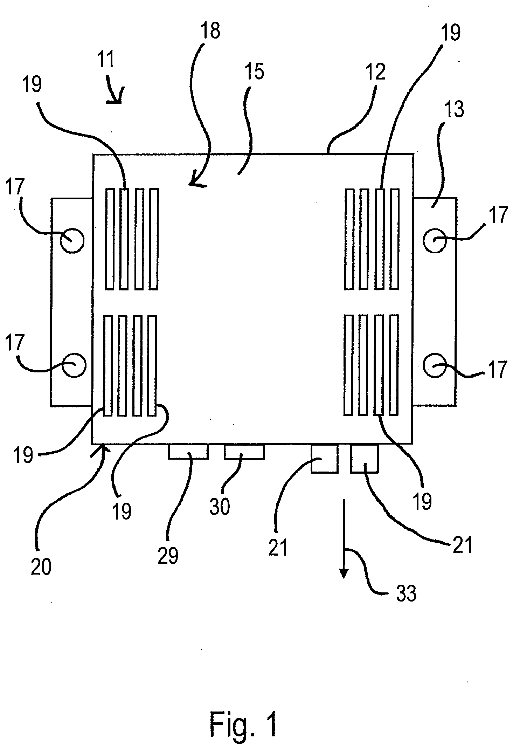

Der in Fig. 1 gezeigte Tunnelüberwachungssensor 11 dient zum Überwachen von Umweltbedingungen in einem Straßen- oder Schienenverkehrstunnel und weist ein Gehäuse 12 auf, welches ein Trägerteil 13 sowie eine halbschalenartige Abdeckung 15 aufweist. An dem Trägerteil 13 sind Befestigungsöffnungen 17 vorgesehen, welche zum Anbringen des Tunnelüberwachungssensors 11 in dem Tunnel, beispielsweise mittels Schrauben, dienen. Die Abdeckung 15 ist zu Wartungszwecken von dem Trägerteil 13 abnehmbar. An der Oberseite 18 des Gehäuses 12 sind mehrere Anordnungen von Lufteintrittsöffnungen 19 vorgesehen. An einer Stirnseite 20 weist das Gehäuse 12 außerdem zwei elektrische Anschlüsse 21 zur Anbindung des Tunnelüberwachungssensors 11 an eine nachgeordnete Komponente eines Überwachungssystems sowie zur Stromversorgung auf.

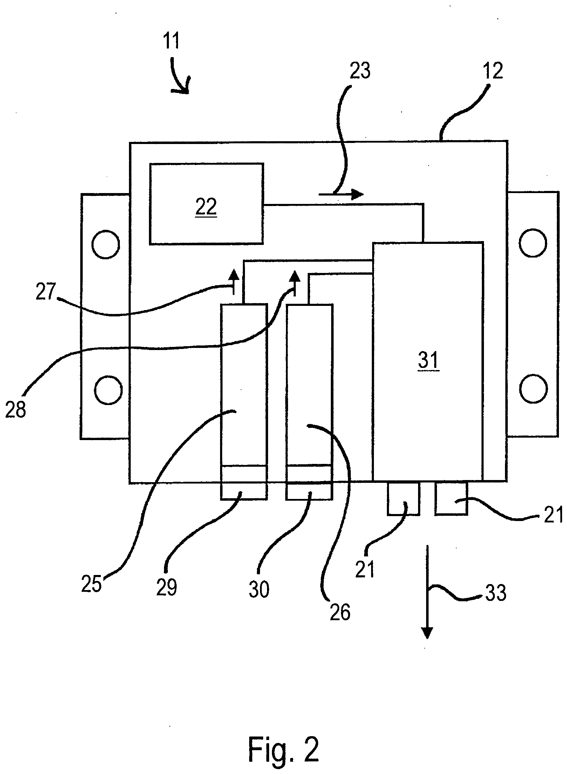

Wie aus der aufgeschnittenen Darstellung gemäß Fig. 2 hervorgeht, weist der Tunnelüberwachungssensor 11 ein Streulicht-Sensormodul 22 auf, welches von der durch die Lufteintrittsöffnungen 19 in das Gehäuse 12 gelangenden Umgebungsluft beaufschlagt wird und ein vorzugsweise digitales Sichttrübungssignal 23 ausgibt. Ferner sind zwei Gaskonzentrations-Sensormodule 25, 26 derart innerhalb des Gehäuses 12 angebracht, dass ihre Messköpfe 29, 30 durch nicht eigens dargestellte Durchführungen des Gehäuses 12 hindurch in den Außenraum des Tunnelüberwachungssensors 11 reichen. Die Gaskonzentrations-Sensormodule 25, 26 weisen nicht dargestellte elektronische Verarbeitungseinrichtungen einschließlich entsprechender Mikroprozessoren und Speichereinrichtungen auf und geben jeweilige, vorzugsweise digitale, Gaskonzentrationssignale 27, 28 aus. Hierbei beziehen sich die beiden Gaskonzentrationssignale 27, 28 auf unterschiedliche Zielgase, beispielsweise auf Kohlenmonoxid einerseits und Stickstoffmonoxid andererseits.

In dem Gehäuse 12 ist auch eine elektronische Verknüpfungseinheit 31 untergebracht, welche sowohl das Sichttrübungssignal 23 als auch die beiden Gaskonzentrationssignale 27, 28 empfängt. Die elektronische Verknüpfungseinheit 31 ist dazu ausgebildet, anhand einer logischen Verschaltung des Sichttrübungssignals 23 und der beiden Gaskonzentrationssignale 27, 28 ein kombiniertes Umweltbedingungssignal 33 zu erzeugen und über die elektrischen Anschlüsse 21 auszugeben. Vorzugsweise gibt die elektronische Verknüpfungseinheit 31 das kombinierte Umweltbedingungssignal 33 über eine digitale Standard-Schnittstelle wie die PROFIBUS-Schnittstelle aus. Bevorzugt erfolgt auch die Kommunikation des Streulicht-Sensormoduls 22 und der Gaskonzentrations-Sensormodule 25, 26 mit der elektronischen Verknüpfungseinheit 31 über digitale Schnittstellen.

Je nach Anwendung kann die logische Verschaltung des Sichttrübungssignals 23 und der Gaskonzentrationssignale 27, 28 relativ einfach sein, d.h. beispielsweise auf einer Summen- und/oder Differenzbildung beruhen, oder es kann eine komplexe Verarbeitung der Eingangssignale stattfinden, beispielsweise unter Verwendung eines Mustererkennungsalgorithmus.

Je nach Anwendung können auch weitere Sensormodule in dem Gehäuse 12 angeordnet sein, z.B. weitere Gaskonzentrations-Sensormodule für andere Zielgase, ein Feuchtesensor und/oder ein Temperatursensor. Gemäß einer nicht dargestellten Ausführungsform weist das Gehäuse 12 zudem einen Eingangsanschluss zum Verbinden eines zusätzlichen externen Sensors mit dem Tunnelüberwachungssensor 11 auf.

Während des Betriebs des Tunnelüberwachungssensors 11 verknüpft die elektronische Verknüpfungseinheit 31 das Sichttrübungssignal 23 mit den beiden Gaskonzentrationssignalen 27, 28 und gibt in Form des kombinierten Umweltbedingungssignals 33 eine Meldung des aktuellen Zustands im Tunnel einschließlich möglicher Störungen und Gefahren aus. Beispielsweise kann das kombinierte Umweltbedingungssignal 33 bei einem Anstieg des Sichttrübungssignals 23 ohne gleichzeitigen Anstieg der Gaskonzentrationssignale 27, 28 das Vorliegen von Nebel oder einer Feinstaubverschmutzung angeben. Sofern zusätzlich ein Feuchtesensor installiert ist, kann anhand des Anstiegs des Feuchtesignals ferner zwischen dem Vorliegen von Nebel und einer Feinstaubverschmutzung unterschieden werden. Sofern zusätzlich ein Temperatursensor installiert ist, kann z.B. das kombinierte Umweltbedingungssignal 33 eine Explosionsgefahr angeben, wenn die Gaskonzentrationssignale 27, 28 kritische Werte erreichen, ein Anstieg des Feuchtesignals und des Temperatursignals jedoch unterbleibt. Zusätzlich könnte das kombinierte Umweltbedingungssignal 33 bei dem Erreichen einer toxischen Gaskonzentration auf eine gefährliche Atmosphäre hinweisen. Bei einem schnellen Anstieg des Sichttrübungssignals 23 und gleichzeitig der Gaskonzentrationssignale 27, 28 sowie gegebenenfalls eines Temperatursignals kann das kombinierte Umweltbedingungssignal 33 einen Brand angeben. Ein Anstieg des Sichttrübungssignals 23 und der Gaskonzentrationssignale 27, 28 ohne gleichzeitigen Anstieg des Feuchtesignals und des Temperatursignals kann hingegen auf einen Stau hinweisen.

Über die PROFIBUS-Schnittstelle kann eine zentrale Steuereinrichtung in der Tunnelwarte das kombinierte Umweltbedingungssignal 33 auslesen, erforderlichenfalls mit weiteren kombinierten Umweltbedingungssignalen 33 von anderen Tunnelüberwachungssensoren 11 in Beziehung setzen und bei erkannten Störungen entsprechende Maßnahmen einleiten. Beispielsweise kann in die Steuerung des Ventilationssystems eingegriffen werden. Ebenso kann bei einem festgestellten Brand ein Feueralarm sowie eine Tunnelsperrung ausgelöst werden.

Bei der bisher beschriebenen Ausführungsform stellt die Verknüpfungseinheit 31 gegebenenfalls auch eine Auswertung der Rohsignale vor der Verknüpfung - beispielsweise eine Digitalisierung - zur Verfügung. Diese Funktion kann jedoch auch in einer gesonderten Auswerteeinheit implementiert sein.

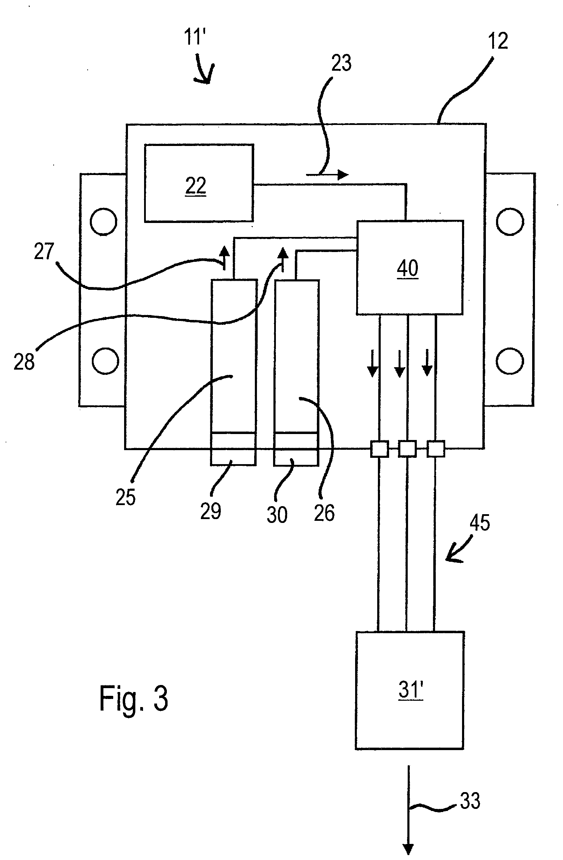

Fig. 3 zeigt eine alternative Ausführungsform eines erfindungsgemäßen Tunnelüberwachungssensors 11', welcher ähnlich aufgebaut ist wie der in Fig. 1 und 2 dargestellte Tunnelüberwachungssensor 11, wobei jedoch die elektronische Verknüpfungseinheit 31' in einer außerhalb des Gehäuses 12 befindlichen übergeordneten Steuerung ausgebildet ist. Insbesondere kann es sich bei dieser externen Verknüpfungseinheit 31' um eine speicherprogrammierbare Steuerung (SPS) handeln. Weiterhin ist bei dem in Fig. 3 dargestellten Tunnelüberwachungssensor 11' eine elektronische Auswerteeinheit 40 vorgesehen, welche bei dieser Ausführungsform in dem Gehäuse 12 angeordnet ist. Die elektronische Auswerteeinheit 40 empfängt das Sichttrübungssignal 23 sowie die beiden Gaskonzentrationssignale 27, 28 und bereitet diese auf, indem sie sie z.B. digitalisiert, sofern die Signale nicht bereits in digitaler Form vorliegen. Prinzipiell könnte auch für jedes der vorhandenen Rohsignale eine eigene Auswerteeinheit vorgesehen sein. Das Sichttrübungssignal 23 und die beiden Gaskonzentrationssignale 27, 28 werden dann in aufbereiteter Form über eine Übertragungsstrecke 45 an die externe Verknüpfungseinheit 31' übermittelt. Diese führt dann eine Verknüpfung wie vorstehend unter Bezugnahme auf Fig. 1 und 2 durch und gibt aufgrund der Verknüpfung das kombinierte Umweltsignal 33 aus.

Dadurch, dass der Tunnelüberwachungssensor 11, 11' ein kombiniertes Umweltbedingungssignal 33 ausgibt, in welchem mehrere unterschiedliche, jedoch zeitgleich erfasste Messgrößen verarbeitet sind, ist in der Tunnelwarte eine besonders zuverlässige Klassifikation des aktuellen Zustands möglich. Insbesondere können Fehlalarme vermieden werden. Durch die Integration mehrerer unterschiedlicher Sensormodule in ein gemeinsames Gehäuse 12 ist die Installation des Tunnelüberwachungssensors 11 vereinfacht. Zudem kann das System auf einfache Weise vor Ort erweitert und mit zusätzlichen Sensorelementen versehen werden, ohne die bestehende Verkabelung zwischen Sensor und Tunnelwarte zu verändern. Dadurch, dass in den einzelnen Sensormodulen 22, 25, 26 Kalibrierparameter in entsprechenden elektronischen Speichereinrichtungen abgelegt sind, entfällt das aufwendige Abgleichen der Sensormodule 22, 25, 26 vor Ort im Rahmen einer Neuinstallation. Aufgrund einer Selbsttestfunktion kann zudem der Ausfall eines Sensormoduls 22, 25, 26 oder des gesamten Tunnelüberwachungssensors 11, 11' frühzeitig erkannt werden.

### Bezugszeichenliste

- 11, 11': Tunnelüberwachungssensor
- 12: Gehäuse
- 13: Trägerteil
- 15: Abdeckung
- 17: Befestigungsöffnung
- 18: Oberseite
- 19: Lufteintrittsöffnung
- 20: Stirnseite
- 21: elektrischer Anschluss
- 22: Streulicht-Sensormodul
- 23: Sichttrübungssignal
- 25: Gaskonzentrations-Sensormodul
- 26: Gaskonzentrations-Sensormodul
- 27: Gaskonzentrationssignal
- 28: Gaskonzentrationssignal
- 29: Messkopf
- 30: Messkopf
- 31, 31': elektronische Verknüpfungseinheit
- 33: kombiniertes Umweltbedingungssignal
- 40: elektronische Auswerteeinheit
- 45: Übertragungsstrecke

## Patentansprüche

1. Tunnelüberwachungssensor (11, 11') zum Überwachen von Umweltbedingungen in einem Tunnel mit
einem ersten Sensormodul (22), welches ein Sichttrübungssignal (23) ausgibt,
einem zweiten Sensormodul (25), welches ein erstes Gaskonzentrationssignal (27) ausgibt,
einem gemeinsamen Gehäuse (12) für das erste und das zweite Sensormodul (22, 25), und
einer vorzugsweise in dem Gehäuse (12) untergebrachten elektronischen Verknüpfungseinheit (31, 31'), welche das Sichttrübungssignal (23) und das Gaskonzentrationssignal (27) empfängt und aufgrund einer Verknüpfung des Sichttrübungssignals (23) und des Gaskonzentrationssignals (27) ein kombiniertes Umweltbedingungssignal (33) ausgibt, wobei
das kombinierte Umweltbedingungssignal (33) bei einem Anstieg des Sichttrübungssignals (23) ohne gleichzeitigen Anstieg des ersten Gaskonzentrationssignals (27) das Vorliegen von Nebel oder einer Feinstaubverschmutzung angibt und/oder
das kombinierte Umweltbedingungssignal (33) bei einem schnellen Anstieg des Sichttrübungssignals (23) und gleichzeitig des ersten Gaskonzentrationssignals (27) einen Brand angibt.

2. Tunnelüberwachungssensor nach Anspruch 1,
**dadurch gekennzeichnet , dass**
das erste Sensormodul (22) und das zweite Sensormodul (25) zum Ausgeben jeweiliger digitaler Signale (23, 27) ausgebildet sind, wobei die elektronische Verknüpfungseinheit (31, 31') aufgrund einer logischen Verschaltung des digitalen Sichttrübungssignals (23) und des digitalen Gaskonzentrationssignals (27) ein kombiniertes digitales Umweltbedingungssignal (33) ausgibt.

3. Tunnelüberwachungssensor nach Anspruch 2,
**dadurch gekennzeichnet, dass**
die logische Verschaltung eine Mustererkennung beinhaltet.

4. Tunnelüberwachungssensor nach Anspruch 2 oder 3,
**dadurch gekennzeichnet, dass**
die elektronische Verknüpfungseinheit (31, 31') das kombinierte digitale Umweltbedingungssignal (33) über eine digitale Standard-Schnittstelle, insbesondere über eine PROFIBUS-Schnittstelle, ausgibt.

5. Tunnelüberwachungssensor nach zumindest einem der vorstehenden Ansprüche,
**gekennzeichnet durch**
wenigstens ein weiteres Sensormodul (26), welches ein weiteres Gaskonzentrationssignal (28) ausgibt, wobei sich das erste Gaskonzentrationssignal (27) und das weitere Gaskonzentrationssignal (28) auf unterschiedliche Zielgase beziehen und die elektronische Verknüpfungseinheit (31, 31') das kombinierte Umweltbedingungssignal (33) aufgrund einer Verknüpfung des Sichttrübungssignals (23), des ersten Gaskonzentrationssignals (27) und des weiteren Gaskonzentrationssignals (28) ausgibt.

6. Tunnelüberwachungssensor nach zumindest einem der vorstehenden Ansprüche,
**dadurch gekennzeichnet, dass**
das zweite Sensormodul (25) an einem Wandabschnitt des Gehäuses (12) angebracht ist, wobei ein Messkopf (29) des zweiten Sensormoduls (25) durch eine Durchführung des Wandabschnitts hindurch in den Außenraum reicht.

7. Tunnelüberwachungssensor nach zumindest einem der vorstehenden Ansprüche,
**dadurch gekennzeichnet, dass**
das erste Sensormodul (22) einen Streulichtsensor umfasst.

8. Tunnelüberwachungssensor nach zumindest einem der vorstehenden Ansprüche,
**gekennzeichnet durch**
wenigstens ein weiteres Sensormodul, welches ein Temperatursignal und/oder ein Feuchtesignal ausgibt, wobei die elektronische Verknüpfungseinheit (31, 31') das kombinierte Umweltbedingungssignal (33) aufgrund einer Verknüpfung des Sichttrübungssignals (23), des ersten Gaskonzentrationssignals (27) und zusätzlich des Temperatursignals und/oder des Feuchtesignals ausgibt.

9. Tunnelüberwachungssensor nach zumindest einem der vorstehenden Ansprüche,
**dadurch gekennzeichnet, dass** die elektronische Verknüpfungseinheit (31, 31') zur Ausgabe eines Fehlersignals ausgebildet ist, welches einen Funktionsstatus des ersten Sensormoduls (22) und/oder des zweiten Sensormoduls (25) angibt.

10. Tunnelüberwachungssensor nach zumindest einem der vorstehenden Ansprüche,
**dadurch gekennzeichnet, dass**
das erste Sensormodul (22) und/oder das zweite Sensormodul (25) eine Speichereinrichtung umfasst/umfassen, in welcher Kalibrierparameter des betreffenden Sensormoduls (22, 25) abgelegt sind.

11. Tunnelüberwachungssensor nach zumindest einem der vorstehenden Ansprüche,
**dadurch gekennzeichnet, dass**
das erste Sensormodul (22) und/oder das zweite Sensormodul (25) einen Mikroprozessor umfasst/umfassen, welcher insbesondere zur Ausführung eines Selbsttests des betreffenden Sensormoduls (22, 25) ausgebildet ist.

12. Tunnelüberwachungssensor nach zumindest einem der vorstehenden Ansprüche,
**dadurch gekennzeichnet, dass**
an dem Gehäuse (12) wenigstens ein Anschluss für ein außerhalb des Gehäuses (12) befindliches Zusatzsensormodul vorgesehen ist.

13. Tunnelüberwachungsverfahren zum Überwachen von Umweltbedingungen in einem Tunnel, bei welchem
mittels eines ersten in einem Gehäuse (12) angeordneten Sensormoduls (22) ein Sichttrübungssignal (23) erzeugt wird,
mittels eines zweiten in dem Gehäuse (12) angeordneten Sensormoduls (25) ein erstes Gaskonzentrationssignal (27) erzeugt wird,
das Sichttrübungssignal (23) und das Gaskonzentrationssignal (27) miteinander verknüpft werden und
aufgrund der Verknüpfung ein kombiniertes Umweltbedingungssignal (33) erzeugt wird, welches
bei einem Anstieg des Sichttrübungssignals (23) ohne gleichzeitigen Anstieg des ersten Gaskonzentrationssignals (27) das Vorliegen von Nebel oder einer Feinstaubverschmutzung angibt und/oder bei einem schnellen Anstieg des Sichttrübungssignals (23) und gleichzeitig des ersten Gaskonzentrationssignals (27) einen Brand angibt.

## Claims

1. A tunnel monitoring sensor (11, 11') for monitoring environmental conditions in a tunnel comprising
a first sensor module (22) which outputs an obscuration signal (23); a second sensor module (25) which outputs a first gas concentration signal (27);
a common housing (12) for the first and second sensor modules (22, 25); and
an electronic logic unit (31, 31') which is preferably accommodated in the housing (12), which receives the obscuration signal (23) and the gas concentration signal (27) and which outputs a combined environmental condition signal (33) on the basis of a link of the obscuration signal (23) and the gas concentration signal (27),
wherein
the combined environmental condition signal (33) indicates the presence of fog or of fine particulate pollution on an increase in the obscuration signal (23) without a simultaneous increase in the first gas concentration signal (27) and/or
the combined environmental condition signal (33) indicates a fire on a fast increase in the obscuration signal (23) and simultaneously in the first gas concentration signal (27).

2. A tunnel monitoring sensor in accordance with claim 1,
**characterized in that**
the first sensor module (22) and the second sensor module (25) are configured for outputting respective digital signals (23, 27), with the electronic logic unit (31, 31') outputting a combined digital environmental condition signal (33) on the basis of a logical connection of the digital obscuration signal (23) and the digital gas concentration signal (27).

3. A tunnel monitoring sensor in accordance with claim 2,
**characterized in that**
the logical connection includes a pattern recognition.

4. A tunnel monitoring sensor in accordance with claim 2 or claim 3,
**characterized in that**
the electronic logic unit (31, 31') outputs the combined digital environmental condition signal (33) via a standard digital interface, in particular via a PROFIBUS interface.

5. A tunnel monitoring sensor in accordance with at least one of the preceding claims,
**characterized by**
at least one further sensor module (26) which outputs a further gas concentration signal (28), with the first gas concentration signal (27) and the further gas concentration signal (28) relating to different target gases and the electronic logic unit (31, 31') outputting the combined environmental condition signal (33) on the basis of a link of the obscuration signal (23), the first gas concentration signal (27) and the further gas concentration signal (28).

6. A tunnel monitoring sensor in accordance with at least one of the preceding claims,
**characterized in that**
the second sensor module (25) is attached to a wall section of the housing (12), with a measuring head (29) of the second sensor module (25) extending into the outside space through a leadthrough of the wall section.

7. A tunnel monitoring sensor in accordance with at least one of the preceding claims,
**characterized in that**
the first sensor module (22) comprises a scattered light sensor.

8. A tunnel monitoring sensor in accordance with at least one of the preceding claims,
**characterized by**
at least one further sensor module which outputs a temperature signal and/or a humidity signal, with the electronic logic unit (31, 31') outputting the combined environmental condition signal (33) on the basis of a link of the obscuration signal (23), the first gas concentration signal (27) and additionally the temperature signal and/or the humidity signal.

9. A tunnel monitoring sensor in accordance with at least one of the preceding claims,
**characterized in that**
the electronic logic unit (31, 31') is configured for outputting an error signal which indicates a functional status of the first sensor module (22) and/or of the second sensor module (25).

10. A tunnel monitoring sensor in accordance with at least one of the preceding claims,
**characterized in that**
the first sensor module (22) and/or the second sensor module (25) comprises/comprise a memory device in which calibration parameters of the respective sensor module (22, 25) are stored.

11. A tunnel monitoring sensor in accordance with at least one of the preceding claims,
**characterized in that**
the first sensor module (22) and/or the second sensor module (25) comprises/comprise a microprocessor which is in particular configured to carry out a self-test of the respective sensor module (22, 25).

12. A tunnel monitoring sensor in accordance with at least one of the preceding claims,
**characterized in that**
at least one connector for an additional sensor module located outside the housing (12) is provided at the housing (12).

13. A tunnel monitoring method for monitoring environmental conditions in a tunnel, in which
an obscuration signal (23) is generated by means of a first sensor module (22) arranged in a housing (12);
a first gas concentration signal (27) is generated by means of a second sensor module (25) arranged in the housing (12);
the obscuration signal (23) and the gas concentration signal (27) are linked to one another; and
a combined environmental condition signal (33) is generated on the basis of the link which
indicates the presence of fog or of fine particulate pollution on an increase in the obscuration signal (23) without a simultaneous increase in the first gas concentration signal (27) and/or
indicates a fire on a fast increase in the obscuration signal (23) and simultaneously in the first gas concentration signal (27).

## Revendications

1. Capteur de surveillance de tunnel (11, 11') pour la surveillance des conditions environnementales dans un tunnel, comportant un premier module capteur (22) qui émet un signal d'opacité (23),
un second module capteur (25) qui émet un premier signal de concentration de gaz (27),
un boîtier commun (12) pour les premier et second modules capteurs (22, 25), et
une unité électronique de combinaison (31, 31') qui est logée de préférence dans le boîtier (12) et qui reçoit le signal d'opacité (23) et le signal de concentration de gaz (27) et qui émet un signal de conditions environnementales (33) combiné sur la base d'une combinaison du signal d'opacité (23) et du signal de concentration de gaz (27),
dans lequel
le signal de conditions environnementales (33) combiné indique la présence de brouillard ou d'un encrassement par poussières fines lorsque le signal d'opacité (23) augmente sans que le premier signal de concentration de gaz (27) n'augmente simultanément, et/ou
le signal de conditions environnementales (33) combiné indique un incendie lorsque le signal d'opacité (23) et simultanément le premier signal de concentration de gaz (27) augmentent rapidement.

2. Capteur de surveillance de tunnel selon la revendication 1,
**caractérisé en ce que**
le premier module capteur (22) et le second module capteur (25) sont réalisés pour émettre des signaux numériques respectifs (23, 27), l'unité électronique de combinaison (31, 31') émettant un signal de conditions environnementales (33) numérique combiné sur la base d'une interconnexion logique du signal d'opacité (23) numérique et du signal de concentration de gaz (27) numérique.

3. Capteur de surveillance de tunnel selon la revendication 2,
**caractérisé en ce que**
l'interconnexion logique inclut une reconnaissance de motifs.

4. Capteur de surveillance de tunnel selon la revendication 2 ou 3,
**caractérisé en ce que**
l'unité électronique de combinaison (31, 31') émet le signal de conditions environnementales (33) numérique combiné via une interface standard numérique, en particulier via une interface PROFIBUS.

5. Capteur de surveillance de tunnel selon l'une au moins des revendications précédentes,
**caractérisé par**
au moins un autre module capteur (26) qui émet un autre signal de concentration de gaz (28), le premier signal de concentration de gaz (27) et l'autre signal de concentration de gaz (28) se référant à différents gaz cibles, et l'unité électronique de combinaison (31, 31') émet le signal de conditions environnementales (33) combiné sur la base d'une combinaison du signal d'opacité (23), du premier signal de concentration de gaz (27) et de l'autre signal de concentration de gaz (28).

6. Capteur de surveillance de tunnel selon l'une au moins des revendications précédentes,
**caractérisé en ce que**
le second module capteur (25) est monté sur une portion de paroi du boîtier (12), une tête de mesure (29) du second module capteur (25) s'étendant vers l'espace extérieur à travers une traversée de la portion de paroi.

7. Capteur de surveillance de tunnel selon l'une au moins des revendications précédentes,
**caractérisé en ce que**
le premier module capteur (22) comprend un capteur de lumière diffuse.

8. Capteur de surveillance de tunnel selon l'une au moins des revendications précédentes,
**caractérisé par**
au moins un autre module capteur qui émet un signal de température et/ou un signal d'humidité, l'unité électronique de combinaison (31, 31') émettant le signal de conditions environnementales (33) combiné sur la base d'une combinaison du signal d'opacité (23), du premier signal de concentration de gaz (27) et en supplément du signal de température et/ou du signal d'humidité.

9. Capteur de surveillance de tunnel selon l'une au moins des revendications précédentes,
**caractérisé en ce que**
l'unité électronique de combinaison (31, 31') est réalisée pour émettre un signal d'erreur qui indique un état de fonctionnement du premier module capteur (22) et/ou du second module capteur (25).

10. Capteur de surveillance de tunnel selon l'une au moins des revendications précédentes,
**caractérisé en ce que**
le premier module capteur (22) et/ou le second module capteur (25) comportent) un moyen de mémorisation dans lequel sont mémorisés des paramètres de calibrage du module capteur (22, 25) correspondant.

11. Capteur de surveillance de tunnel selon l'une au moins des revendications précédentes,
**caractérisé en ce que**
le premier module capteur (22) et/ou le second module capteur (25) comportent) un microprocesseur qui est réalisé en particulier pour effectuer un autotest du module capteur (22, 25) correspondant.

12. Capteur de surveillance de tunnel selon l'une au moins des revendications précédentes,
**caractérisé en ce que**
sur le boîtier (12), il est prévu au moins un raccord pour un module capteur supplémentaire situé à l'extérieur du boîtier (12).

13. Procédé de surveillance de tunnel pour surveiller des conditions environnementales dans un tunnel,
dans lequel
un signal d'opacité (23) est généré au moyen d'un premier module capteur (22) disposé dans un boîtier (12),
un premier signal de concentration de gaz (27) est généré au moyen d'un second module capteur (25) disposé dans le boîtier (12),
le signal d'opacité (23) et le signal de concentration de gaz (27) sont combinés l'un avec l'autre, et
un signal de conditions environnementales (33) combiné est généré sur la base de la combinaison, qui
indique la présence de brouillard ou d'un encrassement par poussières fines lorsque le signal d'opacité (23) augmente sans que le premier signal de concentration de gaz (27) n'augmente simultanément, et/ou
indique un incendie lorsque le signal d'opacité (23) et simultanément le premier signal de concentration de gaz (27) augmentent rapidement.
